(19)

**Europäisches Patentamt**

**European Patent Office**

**Office européen des brevets**

(11) **EP 4 098 286 A1**

(12) **EUROPEAN PATENT APPLICATION**

(43) Date of publication:
**07.12.2022 Bulletin 2022/49**

(21) Application number: **21305722.7**

(22) Date of filing: **31.05.2021**

(51) International Patent Classification (IPC):
**A61L 27/24** (2006.01)   **A61L 27/36** (2006.01)
**A61L 27/38** (2006.01)   **A61L 27/60** (2006.01)
**B33Y 10/00** (2015.01)   **B33Y 80/00** (2015.01)

(52) Cooperative Patent Classification (CPC):
**A61L 27/3834; A61L 27/24; A61L 27/3687;**
**A61L 27/60; B33Y 10/00; B33Y 80/00;**
A61L 2300/608

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **ROQUETTE FRERES**
  **62136 Lestrem (FR)**
• **National University of Singapore**
  **Singapore 119077 (SG)**

(72) Inventors:
• **EE, Pui Lai Rachel**
  **117543 SINGAPORE (SG)**
• **NG, Jian Yao**
  **117543 SINGAPORE (SG)**
• **GOKHALE, Rajeev**
  **138667 SINGAPORE (SG)**
• **LUI, Yuan Siang**
  **138667 SINGAPORE (SG)**

(74) Representative: **Plasseraud IP**
  **66, rue de la Chaussée d'Antin**
  **75440 Paris Cedex 09 (FR)**

(54) **3D-BIOPRINTING OF CELL-LADEN-COLLAGEN GELLAN GUM INTERPENETRATING NETWORK HYDROGEL**

(57)     The present invention relates to a method for preparing cell-laden gellan gum-collagen interpenetrating hydrogel using 3D-bioprinter and a 3D printed hydrogel obtainable thereof, particularly for use in the wound treatment.

**EP 4 098 286 A1**

**Description**

**TECHNICAL FIELD**

The present invention relates to a method for preparing cell-laden collagen-gellan gum interpenetrating network (IPN) hydrogel using 3D-bioprinter and a 3D printed hydrogel obtainable from said method, particularly for use in the wound treatment.

**BACKGROUND OF THE INVENTION**

The human body is able to restore skin integrity after injury with a minimal scar via a complex process involving coagulation and haemostasis, inflammation, proliferation and remodelling. However, the healing process could be interrupted by local or systemic factors, and medical treatment could be required.

The conventional treatment of chronic wounds includes the use of autografts, allografts, or xenografts. However, the availability of autografts is limited and the use of allogeneic or xenogeneic skin grafts may present high risk of graft rejection, limiting their clinical applications.

Bioengineered artificial skin substitutes represent promising therapies for chronic wounds to overcome graft rejection. In particular, cell seeded artificial skin substitutes are particularly of interest as they reduce inflammation and accelerate the healing process (Przekora A; et al. Cells. 2020, 9(7): 1622). However, synthetic skin grafts designed to substitute autografts are not yet widely adopted in the healthcare field given the need for laboratory-based fabrication process which is not adapted for healthcare application.

The inventors have previously developed a biocompatible gellan gum-collagen IPN hydrogel allowing long-term cell growth (WO2020/225336) which enhance early wound closure, reduced inflammation, and promote complete skin regeneration. However, the construction of this hydrogel involves labor-intensive and time-consuming cell culture processes which is not optimal for healthcare application.

3D-bioprinting technology is a promising technology in tissue engineering to produce artificial skin substitutes. Although, several processes for an extrusion-based 3D-bioprinting of hydrogel constructs has been disclosed, it remains a need to develop 3D printing process to produce complex hydrogel such as interpenetrating polymer network which required precise temperature control to maintain their gelation properties.

**SUMMARY OF THE INVENTION**

The inventors transposed the temperature dependent gelation protocol into one-step 3D bioprinting procedure to achieve gellan gum-collagen interpenetration and allow the 3D-biofabrication of cell-laden collagen-gellan gum IPN hydrogel. In particular, they developed bioinks with adequate shear-thinning property required for them to pass through the bore of the ejector tip at high shear, as well as return to its original viscosity once the shear force is removed. The rapid return of viscosity allows the bioink deposited on the printbed to preserve structural integrity.

The present invention relates to a method for preparing cell-laden collagen- gellan gum interpenetrating network (IPN) hydrogel by 3D bioprinting, the method comprising:

   a) preparing a first bioink by dissolving gellan gum with a viscosity enhancer in a solvent, preferably by heating the solution at a temperature above hydration temperature and by lowering the temperature,
   b) preparing a second bioink by suspending stem cells in collagen solution,
   c) depositing the first bioink on a support media,
   d) depositing the second bioink into the first bioink,
   e) heating the bioprinted product obtained from the deposition of the first and second bioinks of step c) and d), preferably to 37°C or above to achieve collagen fibrillogenesis,
   f) adding an ionic crosslinking agent, preferably $MgCl_2$ solution to cross-link gellan gum to obtain a cell-laden collagen-gellan gum IPN hydrogel.

In another aspect, the present invention relates to a 3D bioprinted cell-laden collagen-gellan gum IPN hydrogel comprising a viscosity enhancer such as a pregelatinized starch derived from potato.

The present invention also relates to an artificial skin graft comprising the 3D printed hydrogel film as described above. Finally, the present invention relates to a kit comprising a first bioink comprising gellan gum and a viscosity enhancer and a second bioink comprising cell laden collagen solution.

## LEGEND FIGURES

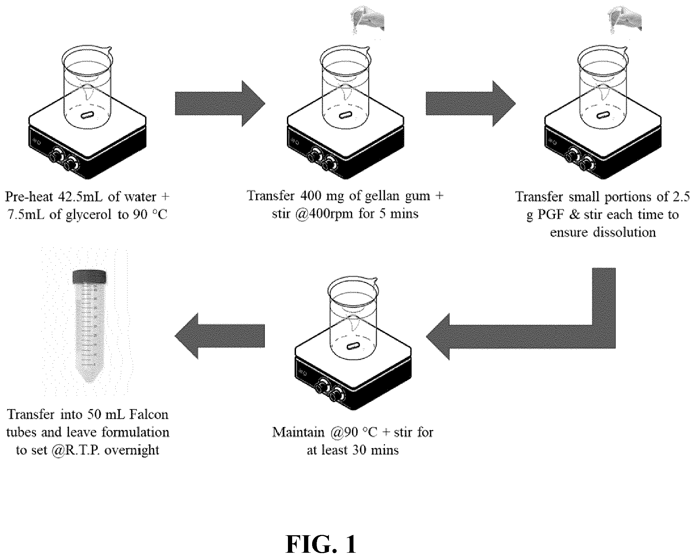

**Figure 1:** Formulation schema for incorporating glycerol and pregelatinized starch (also referred as "PGS" in the instant disclosure) into a gellan gum precursor solution. The glycerol-water solvent mixture was preheated to 90 °C before gellan gum powder was added. The suspension was kept at this temperature and stirred for 5 minutes until a homogeneous solution is obtained. PGS was then added via geometric dilution. After all the PGS powder was dissolved, the bioink was maintained at 90 °C and continuously stirred for 30 minutes. After which, the bioink was allowed to cool at room temperature and pressure (R.T.P.) and left overnight to set.

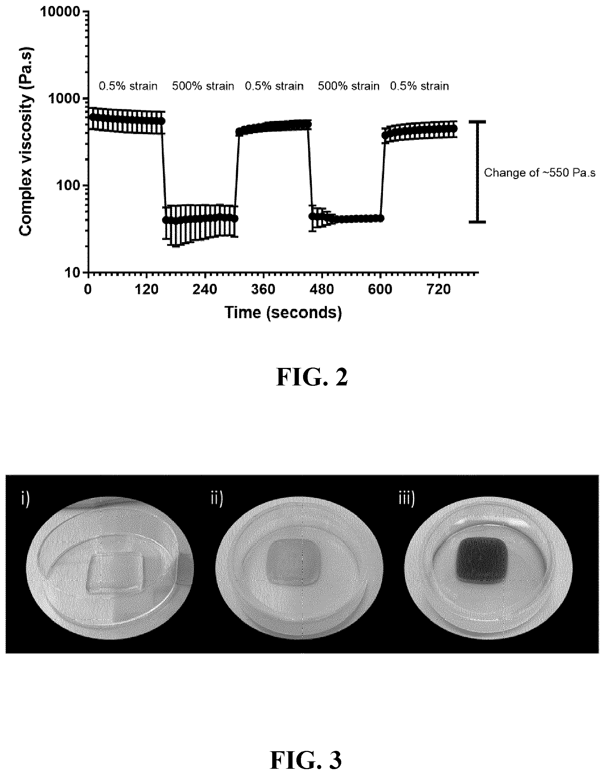

**Figure 2:** Step-strain rheological measurements of glycerol-PGS-gellan gum composite. Complex viscosity (Pa.s) values of the composite bioink were obtained through iterations of non-deformatory (0.5% shear strain) and deformatory (500% shear strain) shear forces. Within 10 seconds, a change of an average of ~550 Pa.s of complex viscosity was registered for the bioink upon change in shear forces. The measurements were performed at 25 °C. For the displayed graphic, mean (n = 3) and SD are shown.

**Figure 3:** Top view of 3D-printed gellan gum-collagen IPN hydrogel films (i) immediately after 3D-printing, (ii) 72 hours after incubation, and (iii) 21 days after incubation in cell culture media.

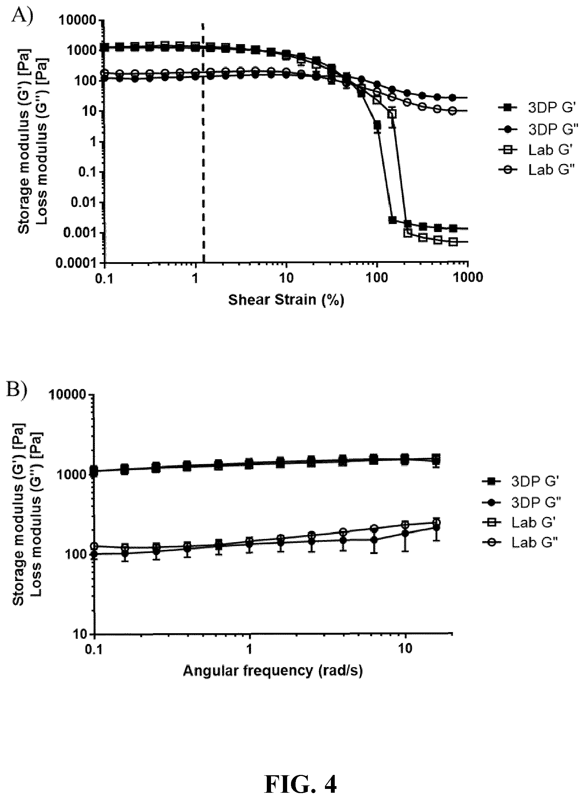

**Figure 4:** Rheometry of manually fabricated and 3D-printed gellan gum-collagen IPN hydrogel films. (A) Amplitude sweeps were conducted to identify the hydrogels' limit of linear viscoelasticity (LVE). (B) Frequency sweeps were conducted, within the range of LVE, to determine the hydrogels' viscoelastic properties as a function of shear frequency. Plots of storage modulus (G') [Pa] and loss modulus (G") [Pa] against shear strain (%) or frequency (rad/s) are presented as mean $\pm$ SD, n = 3.

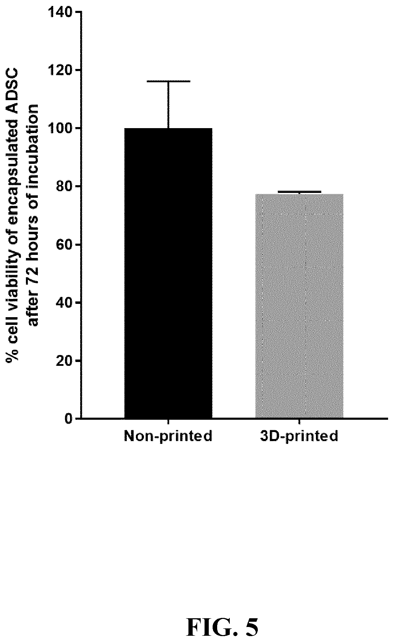

**Figure 5:** Cell viability of ADSC encapsulated within 3D-bioprinted and manually fabricated IPN hydrogel films, determined after 72 hours of incubation in basal cell culture conditions. Cell viability was quantified using MTS assay, percentage cell viabilities were expressed against that of ADSC encapsulated within manually fabricated hydrogel films. Data is presented with mean $\pm$ SD, n = 3.

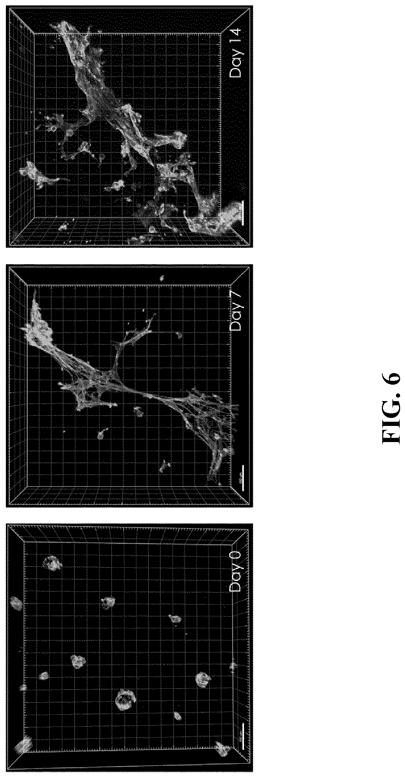

**Figure 6:** Three dimensional (3D) confocal images of encapsulated ADSC, F-actin (cytoskeleton) of the cells was stained green with Phalloidin-iFluor 488 reagent. Z-stacked images were captured with CLSM using Zeiss LSM 710 to analyze the change in cell morphology in the entirety of the cell-laden hydrogels at referred time points. Cell adhesion to, and cell spread within, IPN hydrogels could be observed in all 3 dimensions. (Scale bar: 100 $\mu$m)

## DETAILED DESCRIPTION OF THE INVENTION

### Method for preparing 3D bioprinted hydrogel

The inventors developed a method for preparing by 3D-bioprinting a cell-laden collagen-gellan gum IPN hydrogel.

The expression "interpenetrating polymer network (IPN)" classically refers to a polymer structure comprising two or more networks which are at least partially interlaced on a polymer scale, and preferably not covalently bonded to each other. As opposed to a mixture of two or more pre-formed polymer networks, an IPN typically appears as a monophasic structure.

The term "hydrogel" as used herein refers to hydrophilic macromolecular networks which are produced by chemical or physical crosslinking of soluble polymers. Preferably, the hydrogels have the capability to absorb and retain a high content of water, and/or have a visco-elastic character and/or facilitate the transport of oxygen, nutrients and waste.

"Bioprinting" refers to methods that employ living cells. It is preferably performed by 3D printing. In addition to the cells, it can include growth factors, and biomaterials to fabricate biomedical parts that maximally imitate natural tissue characteristics. Generally, 3D bioprinting utilizes the layer-by-layer method to deposit materials known as bioinks to create tissue-like structures that are later used in medical and tissue engineering fields.

The 3D printer may be any 3D printer such as an inkjet printer, a robotic dispensing printer or an extrusion-based printer. Preferably, the 3D printer is an extrusion-based printer.

Extrusion-based printing is a well-known technique in the field of 3D printing which entails extruding or forcing a continuous stream of melted solid material (e.g. hot melt extrusion techniques) or viscous liquid (e.g. cold extrusion techniques) through an orifice such as a nozzle or syringe. Extrusion-based 3D printing can be pneumatic driven, piston driven, and screw driven. Pneumatic extrusion used pressurized air to force bioink through a depositing agent. Piston driven extrusion utilizes a piston connected to a guide screw. The linear motion of the piston squeezes material out of the nozzle. Screw driven extrusion uses an auger screw to extrude material. The rotational motion forces the material down and out of the nozzle.

In a preferred embodiment, said 3D bioprinter used according to the present disclosure is extrusion-based 3D printer, preferably cold-extrusion based 3D printer. It is preferably a pneumatic driven extrusion 3D printer. The bioprinter apparatus can be of any commercially available type, such as the 3D Bioprinter Cellink BioX bioprinter (Cellink Life sciences).

In a first aspect, the present disclosure relates to a method of preparing a hydrogel comprising an interpenetrating network comprising cell-laden collagen and gellan gum by using 3D printing.

The inventors developed two bioinks with adequate shear-thinning property required for them to pass through the bore of the ejector tip at high shear, as well as return to its original viscosity once the shear force is removed.

"Bioink" is defined as cell compatible material that can be 3D printed and provides a support to cells or comprises cells. Bioinks may be extruded through a needle and then can be gelled or solidified.

In one embodiment, the present disclosure relates to a first bioink comprising gellan gum, preferably low-acyl gellan gum and a viscosity enhancer.

Gellan gum is a polysaccharide secreted by *Sphingomonas paucimobilis* that was initially described by Moorhouse R. et al. 1981. ACS symposium Series. No. 150, p. 111. It consists of tetrasaccharide repeat units containing $\beta$-D-glucose, $\beta$-D-glucuronic acid and $\alpha$(L-rhamnose) monomers in the molar ratio 2:1:1. It exists commonly in two formulations: one with a high acyl content which is the raw product secreted by the bacteria, and another with low acyl content due to processing, which is the wider known. Gellan gum includes, but is not limited to, low-acyl gellan gum, high-acyl gellan gum, chemically modified gellan gum, or any mixture of these gellan gum polymers. Preferably, said gellan gum is a low-acyl gellan gum.

In an embodiment for better results, the concentration of the gellan gum in the first bioink may be between 0.1 % and 10 % (w/v), preferably between 0.2 % and 5 % (w/v), more preferably between 0.2 % and 2.5 % (w/v), or between 0.2% and 1 % (w/v). Even more preferably, the concentration of gellan gum in the bioink is equal to about 0.8% (w/v).

According to the present disclosure, the bioink comprises a viscosity enhancer to modulate the final viscosity of the bioink formulation for ensuring the extrusion of the bioink during 3D bioprinting process.

In particular, the first bioink further comprises a viscosity enhancer to obtain bioink with adequate shear-recovery property, in particular to achieve a shear strain recovery between 400 to 600 Pa.s. The said shear strain recovery measurement was performed using a rheometer (MCR 302) at a constant shear frequency of 1 Hz. The measurement of complex viscosity was conducted across three iterations of non-deformatory and deformatory of 0.5% and 500% shear strains, respectively. A total of 15 complex viscosity measurements at each shear strain were performed, each at an interval of 10 seconds. Complex viscosity values were calculated from an average of three samples, all measured at 25°C.

Examples of viscosity enhancers that may be included in the bioink include, but are not limited to: hydroxypropylcellulose, hypromellose, methylcellulose, hydroxyethylcellulose, and like water-soluble cellulose; crystalline cellulose, low-substituted hydroxypropyl cellulose, carmellose, carmellose calcium, carmellose sodium, croscarmellose sodium, carboxymethylethylcellulose, ethylcellulose, hypromellose phthalate, hydroxypropylmethylcellulose acetate phthalate, cellulose acetate, cellulose acetate phthalate, and like water-insoluble cellulose; polyvinyl pyrrolidone, polyethylene oxide, carboxyvinyl polymer, polyvinyl alcohol (partially or fully saponified), and like water-soluble synthetic polymers; crospovidone, polycarbophil, polycarbophil calcium, aminoalkyl methacrylate copolymer E, aminoalkyl methacrylate copolymer RS, methacrylic acid copolymer L, methacrylic acid copolymer S, methacrylic acid copolymer LD, dry methacrylic acid copolymer LD, polyvinylacetal diethylaminoacetate, and like water-insoluble synthetic polymers; wheat starch, rice starch, corn starch, potato starch, partially pregelatinized starch, pregelatinized starch, dextrin, $\alpha$-cyclodextrin, $\beta$-cyclodextrin, maltodextrin, isomalt, hydroxypropyl starch, sodium carboxymethyl starch, pullulan, and like starches; gum arabic, gum arabic powder, agar, agar powder, gelatin, purified gelatin, chitosan, xanthan gum, pectin, sodium alginate, locust bean gum, guar gum, and like natural polymer compounds; stearic acid, monoglycerol stearate, carnauba wax, stearyl alcohol, cetanol, macrogol 1500, macrogol 4000, macrogol 6000.

In a preferred embodiment, said viscosity enhancer is a pregelatinized starch. The starch molecules may be derived from any plant source of starch, especially such as corn, potato, oat, rice, tapioca, pea, sorghum, barley or wheat. Pregelatinized starches can be made by creating a starch paste that is then heated to its gelatinization temperature. Once gelled, the mixture is dried on a drum dryer and ground into a powder. In a preferred embodiment, said pregelatinized starch is derived from potato.

Said pregelatinized starch is preferably cross-linked. Cross-linking can be achieved for example by esterification with various reagents such as for example acetic adipic anhydride, phosphorous oxychloride and sodium trimetaphosphate. Preferably, pregelatinized starch is cross-linked with sodium trimetaphosphate. In a preferred embodiment, said viscosity enhancer is pregelatinized starch derived from potato (CAS No. 55963-33-2) represented for example by the product sold under the name PREGEFLO® PI10 modified starch.

Typically, first bioink of the present disclosure contains between 1 and 20 % (w/v) of viscosity enhancer, preferably between 1 and 10% (w/v), more preferably between 2 and 10% (w/v), again more preferably about 5% (w/v) of viscosity enhancer. In a preferred embodiment, said viscosity enhancer is pregelatinized starch, preferably as described above.

In a preferred embodiment, said first bioink can comprise a hydrophilic plasticizer. This is particularly useful in order to decrease hydrogel's brittleness and/or to make the material ductile and/or to make the material stretchable when 3D printed into the form of a film. Said hydrophilic plasticizers include, but are not limited to diesters derived from dicarboxylic acids (e.g. sebacic acid, azelaic acid) or from ethylene glycol and propylene glycol, citric acid (tributylcitrate, triethylcitrate) or glycerol (triacetin, tributyrin). In a preferred embodiment, the first bioink as described above further comprises glycerol. Typically, first bioink of the present disclosure may contain between 1 and 30 % (v/v) of hydrophilic plasticizer, preferably between 10 and 20% (v/v), more preferably about 15% (v/v), again more preferably about 15% (v/v) of hydrophilic plasticizer such as glycerol.

The first bioink according to the present disclosure can be prepared by dissolving gellan gum with a viscosity enhancer as described above in a solvent. In particular, the dissolution can be realized by heating said solution comprising gellan gum and viscosity enhancer at a temperature above the hydration temperature, typically above 70°C, preferably, above 80°C, more preferably of about 90°C, until an homogeneous solution is obtained (e.g. during at least 20 - 30 minutes) and by lowering the temperature, preferably to a temperature selected from 4°C to 40°C, more preferably between 36°C to 38°C. The chains of gellan gum are uncoiled above hydration temperature and double helices are then formed by lowering temperature.

According to the disclosure, a suitable solvent is an aqueous solution, preferably water, a cell culture media, an aqueous saline solvent, or mixtures thereof. The water used according to the invention may be purified water or sterilized water.

In particular, a hydrophilic plasticizer, such as glycerol can be added in said solvent before to be heated above hydration temperature.

In a preferred embodiment, said gellan gum is dissolved in solvent by pre-heating the solvent (e.g. water) above 70°C, preferably at about 90°C, optionally with glycerol. Gellan gum and viscosity enhancer (e.g. pregelatinized starch, preferably as described before) are then added one after the other in the solvent. Said solution is then heated above 70°C, preferably at about 90°C until a homogeneous solution is obtained, preferably during at least 20 min, preferably during about 30 min. The solution is then cooled, preferably to a temperature selected from 4°C to 40°C, more preferably between 36°C to 38°C and can be maintained at this temperature until further use.

In another embodiment, the present disclosure relates to a second bioink comprising cells and collagen solution, preferably stem cell and neutralized type I collagen solution.

Collagen is the main structural protein in the extracellular matrix found in the body's various connective tissues. Collagen consists of amino acids bound together to form a triple helix of elongated fibril known as a collagen helix. It is mostly found in connective tissue such as cartilage, bones, tendons, ligaments, and skin. The collagen may be selected from native collagens such as types I, II or III native collagens, atelopeptide collagen and regenerated collagen. In a preferred embodiment, the collagen is type I collagen. The most common sources of collagen I are rat tail, bovine skin and tendons or porcine skin.

In a preferred embodiment, the second bioink comprises less than 0.2% (w/v), preferably from 0.2 to 0.1% (w/v) of collagen, more preferably about 0.1% (w/v) of collagen. Preferably the second bioink comprises less than 0.2% (w/v) of type I collagen, more preferably from 0.2 to 0.1% (w/v), more preferably about 0.1% (w/v) of type I collagen.

In a preferred embodiment, said collagen is neutralized collagen, preferably neutralized type I collagen. Type I collagen solution is for example neutralized with NaOH solution. Neutralized collagen solution is a solution with a pH comprised between 6 and 8, preferably equal to about 7.

Said collagen solution (e.g. type I collagen solution) is preferably prepared by dissolving collagen (e.g. type I collagen) in a buffer solution used for cell culture application which maintains a constant pH and which is isotonic and non-toxic to most cells. In a preferred embodiment, said buffer solution is phosphate-buffered saline (PBS) which is commonly used in cell culture. PBS is a water-based salt solution containing disodium hydrogen phosphate, sodium chloride and in some formulations, potassium chloride and potassium dihydrogen phosphate. Said collagen solution is preferably neutralized by adding basic solution such as NaOH solution.

The second bioink may comprise one or more types of cells. Cells that can be added to the collagen solution, preferably neutralized type I collagen solution as described above can be any cells including stem cells, in particular stem cells which are not derived from human embryonic stem cells, induced pluripotent stem cells, progenitor cells, and differentiated cells. The cells can derive from embryonic, foetal, or adult tissues. The cells can be unipotent, oligopotent, multipotent, or pluripotent. In some embodiments, the cells are adult stem cells.

The cells can be allogeneic or autologous, preferably autologous. In preferred embodiments, the cells include mesenchymal stem cells (MSCs). The composition can contain a single cell type, such as MSCs. However, in some embodiments, the composition contains two or more different types of cells, i.e., cells of two or more different lines. The cells can be animal cells, such as human cells. In a preferred embodiment, said cells are adipose-derived stem cells (ADSCs). Typically, ADSCs are isolated from stromal vascular fraction (SVF), which is typically obtained by digestion of adipose tissue with collagenase, followed by few cycles of centrifugation. Cells may be purchased from a manufacturer and cultured in culture media under culture condition well-known in the art to be expanded before to be harvested and resuspended in the collagen solution.

Cells are then resuspended in the collagen solution to obtain the second bioink. As the cells are resuspended in the collagen solution, the collagen solution is preferably sterile and may comprise antibiotics or other agents that promote a sterile condition. In a preferred embodiment, said cells are adult stem cells such as ADSCs. Cells are preferably resuspended in type I collagen solution at a cell concentration of at least $10^4$ cells/mL, preferably at least about $10^5$ cells/mL, more preferably at least about $10^6$ cells/mL. The second bioink is preferably kept at a temperature below 37°C, preferably selected from 10°C to 30°C, still preferably from 18 to 25°C, still preferably from 19 to 25°C, for example equal to about 20°C, before being deposited into the first bioink to prevent fibrillogenesis from taking place.

The present disclosure also relates to a method for preparing cell-laden collagen-gellan gum IPN hydrogel by 3D bioprinting comprising preparing the first and second bioink as described above, depositing said first bioink on a support media, depositing said second bioink into the first bioink and crosslinking the gellan gum by adding a crosslinking agent.

Thus, after preparing the first and second bioinks as described previously, the first bioink is then deposited on a support media with 3D bioprinter. In some embodiments, the support media is a sterile culture dish, preferably plastic or glass dish.

Preferably, the printing pressure of the first bioink during the 3D bioprinting is selected from 1 to 200 kPa, and is preferably below 50 kPa. It is still preferably selected from 5 to 30 kPa, still preferably from 10 to 25 kPa, still preferably from about 15 to about 20 kPa. The first bioink is preferably at a temperature selected from 4°C to 40°C, still preferably from 10 to 40°C, still preferably from 20 to 40°C, still preferably from 30 to 40°C, still preferably from 35 to 39 °C, still preferably equal to about 37°C, before to be deposited on a surface.

Then, the second bioink is deposited into the first bioink. Indeed, the 3D bioprinter allows the second bioink to be sequentially deposited at the same location than the first bioink. In particular, the printing tip of the second printhead was dipped into the deposited first bioink to facilitate interpenetration. The second bioink is preferably maintained at a temperature below 37°C, preferably selected from 10 to 30°C before being deposited into the first bioink to prevent fibrillogenesis from taking place. It is preferably at room temperature. In other words, no specific temperature is applied *via* the corresponding printhead. This temperature is thus preferably at a temperature selected from 18 to 25°C, still preferably from 19 to 25°C, for example equal to about 20°C. The printing pressure during the 3D bioprinting of the second bioink is preferably selected from 1 to 200 kPa, and preferably below 50 kPa. It is still preferably selected from 1 to 10 kPa, still preferably from 2 to 9 kPa, still preferably from 4 to 8 kPa, still preferably from about 5 to about 7 kPa, since said second bioink contains cells.

According to the method of the present disclosure, the 3D bioprinted product obtained from the deposition of the first and second bioinks is heated in order to achieve collagen fibrillogenesis. Preferably, this heating step is performed by heating the print-bed of the 3D printer. Preferably, the temperature is 37°C or above, still preferably from 37 to 40°C. It is for example equal to about 37°C. preferably, this heating steps is performed for at least 20 minutes, preferably during 20 to 30 minutes.

According to the present disclosure, the method finally comprises a step of crosslinking the gellan gum to obtain a cell-laden gellan gum collagen interpenetrating hydrogel. The crosslinking is achieved after the printing of the 3D structure, preferably by adding a cross-linking agent. In a preferred embodiment, the crosslinking agent is an ionic cross-linking agent such as a cation, more preferably a divalent cation. Said divalent cation can be selected from the group consisting of: $Ca^{2+}$, $Mg^{2+}$, $Mn^{2+}$, $Cu^{2+}$, $Sr^{2+}$, $Zn^{2+}$, $Ra^{2+}$, $Be^{2+}$, or form a mixture thereof. It is preferably $Mg^{2+}$. In a preferred embodiment, a culture media comprising said ionic cross-linking agent, such as $MgCl_2$ enriched culture media is added to cross-link the gellan gum.

Numerous culture media are available commercially and are well-known to the person skilled in the art. This medium may be a minimum medium particularly comprising mineral salts, amino acids, vitamins and a carbon source essential to cells and a buffer system for regulating pH. The basal medium able to be used in the method according to the invention includes, for example, but are not limited to, DMEM/F12 medium, DMEM medium, RPMI medium, Ham's F12 medium, IMDM medium and KnockOut™ DMEM medium (Life Technologies).

Preferably, the concentration of said divalent cation in culture medium, preferably Mg2+, is from 0.01% (w/v) to 0.03% (w/v). It is more preferably equal to about 0.02% (w/v).

Said cell-laden collagen gellan gum IPN hydrogel can thereafter be maintained under physiological conditions in culture media for cell culture.

**Bioprinted hydrogel**

In another aspect, the present disclosure relates to a 3D printed hydrogel prepared or obtainable by the method as described above.

In particular the present disclosure relates to a 3D printed hydrogel film comprising a cell-laden collagen and gellan gum IPN and a viscosity enhancer such as a pregelatinized starch, as described above. Preferably, said 3D printed hydrogel film comprises between 1 and 20 % (w/v), still preferably between 1 and 10 % (w/v), more preferably about 5%, still preferably about 2.5% (w/v) of said pregelatinized starch. In a preferred embodiment, said 3D printed hydrogel comprises between 0.1 and 10 % (w/v), preferably 0.8%, still preferably about 0.4% (w/v) of gellan gum, preferably low-acyl gellan gum. Said 3D printed hydrogel film preferably comprises less than 1 mg/mL of collagen, more preferably less than 0.5 mg/mL of collagen.

In a more preferred embodiment, said 3D printed hydrogel comprises glycerol. Preferably, said 3D printed hydrogel comprises between 1 and 30 % (v/v) glycerol, still preferably between 10 and 20 % (v/v) glycerol, more preferably about 15% (v/v) glycerol, still preferably about 7.5% (v/v).

In a preferred embodiment, the bioprinted hydrogel has a cell concentration of at least $10^4$ cells/ml, preferably of at least $10^5$ cells/ml, still preferably of at least $10^6$ cells/ml, for example of at least $10^7$ cells/ml, or of at least $10^8$ cells/ml. Typically, the cell concentration in the tissue pattern is no higher than about $10^8$ cells/ml, or no higher than about $10^9$ cells/ml. Said cells are preferably stem cells, more preferably adult stem cells, again more preferably adipose derived stem cells.

**An artificial skin graft**

In another aspect, the disclosure relates to an artificial skin graft comprising the bioprinted hydrogel as described above. The artificial skin graft allows the 3D bioprinted hydrogel of the disclosure to be placed in contact with the wound.

The artificial skin graft comprising the bioprinted hydrogel as disclosed above may comprise one or more active therapeutic or antimicrobial agents. Said active or antimicrobial agents may be added within the hydrogel or added separately in the artificial skin graft.

The artificial skin graft according to the disclosure may comprise an acceptable topical carrier. The term "acceptable topical carrier" encompasses both pharmaceutically-acceptable carriers and cosmetically-acceptable carriers, and encompasses substantially non-irritating compatible components which are suitable for delivering the active components to the skin. The term "compatible," as used herein, means that the components of the carrier must be capable of being commingled with the hydrogel, in a manner such that there is no interaction which would substantially reduce the efficacy of the hydrogel during use for treating wounds. These carriers must, of course, be of sufficiently high purity and sufficiently low toxicity to render them suitable for chronic topical administration to the skin of humans or lower animals.

**Therapeutic use**

Bioprinted hydrogel or the artificial skin graft according to the disclosure may be used for the treatment of wounds in a subject in need thereof.

The term "subject" or "patient" as used herein, refers to mammals. Mammalian species that can benefit from the disclosed methods of treatment include, but are not limited to, humans, non-human primates such as apes, chimpanzees, monkeys, and orangutans, domesticated animals, including dogs and cats, as well as livestock such as horses, cattle, pigs, sheep, and goats, or other mammalian species including, without limitation, mice, rats, guinea pigs, rabbits, hamsters, and the like.

The term "wound" is used to refer broadly to injuries to the skin and mucous membrane. In particular the term "wound" refers to a damaged area of the body, such as a cut, hole and burn in the skin or the flesh. The wound can be a chronic or an acute wound. An acute wound may be an injury to the skin, mucous membrane that occurs suddenly for example following an accident or surgical injury. A chronic wound is a wound that does not heal in an orderly and timely manner. Chronic wounds include but are not limited to venous and arterial ulcers, diabetic ulcers, pressure ulcers, radiation poisoning, infection or ischemia.

As used herein, the term "treatment", "treat" or "treating" refers to any act intended to ameliorate the status of patients. In certain embodiments, such term refers to the amelioration of the wound healing. As used here the term "wound healing" refers to an intricate process in which the skin or mucous membrane repairs itself after injury.

In a related aspect, the invention pertains to the use of the bioprinted hydrogel or the artificial skin graft of the disclosure in the preparation of a medicament for use in the treatment of a wound.

In a further aspect, the present invention relates to a method of treating wound in a subject in need thereof that comprises administering a therapeutically efficient amount of a hydrogel or an artificial skin graft according to disclosure to the surface of a wound of a subject. In a preferred embodiment, said administration is topical administration which includes application onto the skin and mucous membranes.

This topical administration can be as a single dose or as repeated doses given at multiple designated intervals. It will readily be appreciated by those skilled in the art that the preferred dosage regimen will vary with the type and severity of the injury being treated.

By "therapeutically effective amount" refers to an amount effective, at dosages and for periods of time necessary to achieve the desired therapeutic result such as accelerated wound healing. The therapeutically effective amount of the hydrogel or the artificial skin graft that comprises it may vary according to factors such as the wound type (mechanical or thermal, full or partial thickness, etc.), the size of the wound, the wound's depth (if full thickness), the absence or presence of infection, time elapsed since the injury's infliction, and the age, physical condition, existence of other disease states, and nutritional status of the patient.

Dosage regimens may be adjusted to provide the optimum therapeutic response. A therapeutically effective amount is also typically one in which any toxic or detrimental effect of the product or pharmaceutical composition is outweighed by the therapeutically beneficial effects.

**Kit**

In another aspect, the present disclosure relates to a kit comprising a first bioink and a second bioink as described above. Preferably, the kit comprises containers each comprising one or more compounds at a concentration or in an amount that facilitates the reconstitution and/or the use of the bioinks and the implementation of the method according to the disclosure.

In particular, said first bioink comprises gellan gum, preferably low-acyl gellan gum and viscosity enhancer. In preferred embodiment, the concentration of the gellan gum in the first bioink is between 0.1 % and 10 % (w/v), still preferably between 0.2 % and 5 % (w/v), more preferably between 0.2 % and 2.5 % (w/v), or between 0.2% and 1 % (w/v). Even more preferably, the concentration of gellan gum is equal to about 0.8% (w/v).

In a preferred embodiment, said viscosity enhancer is a pregelatinized starch, preferably as described before. It is preferably cross-linked. It is preferably derived from potato (CAS No. 55963-33-2).

Typically, first bioink of the present disclosure contains between 1 and 20 % (w/v) of viscosity enhancer, preferably between 1 and 10% (w/v), more preferably between 2 and 10% (w/v), again more preferably about 5% (w/v) of viscosity enhancer. Preferably, the viscosity enhancer is pregelatinized starch, said pregelatinized starch being preferably as described before.

In a preferred embodiment, said first bioink can comprise a hydrophilic such as glycerol, preferably contains between 1 and 30 % (v/v) of hydrophilic plasticizer, preferably between 10 and 20% (v/v), more preferably about 15% (v/v) of hydrophilic plasticizer, again more preferably about 15% (v/v) of hydrophilic plasticizer such as glycerol.

The second bioink comprises collagen and cells, preferably stem cells and neutralized type I collagen. The second bioink may comprise one or more types of cells as described above, preferably adult stem cells such adipose derived stem cells.

In a preferred embodiment, the second bioink comprises less than 0.2% (w/v), preferably from 0.2 to 0.1% (w/v) of type collagen, more preferably about 0.1% of collagen. Preferably the second bioink comprises less than 0.2% of type I collagen, more preferably from 0.2 to 0.1%, more preferably about 0.1% of type I collagen. In a preferred embodiment, said collagen is neutralized collagen, preferably neutralized type I collagen.

The kit may also comprise instructions indicating the methods for preparing and/or using the bioinks for preparing 3D bioprinted hydrogel according to the method of the disclosure.

The present invention also relates to the use of the kit according to the invention for preparing 3D bioprinted hydrogel according to the methods of the invention. Other features and advantages of the invention will become more apparent upon reading the following examples given by way of non-limiting illustration.

**EXAMPLES**

**1. Materials**

Low-acyl gellan gum (Gelzan™ CM, G1910), magnesium chloride (MgCl2, 208337), sodium hydroxide (NaOH, S8045), low-glucose Dulbecco's modified Eagle's medium (DMEM, D5523), Accutase® solution (A6964), penicillin/streptomycin (P4333), paraformaldehyde (PFA, P6148), and glycerol (G5516) were purchased from Sigma-Aldrich (St Louis, MO, USA). Type-1 rat tail collagen (SC-136157) was procured from Santa Cruz Biotechnology, Inc. (Dallas, TX, USA). Fetal Bovine Serum (FBS, SV30160.03) and trypan blue were obtained from Hyclone (South Logan, UT, USA). Ultrapure grade phosphate buffered saline (10x PBS) was procured from Vivantis Technologies Sdn Bhd (Subang Jaya, Selangor Darul Ehsan, Malaysia). CellTiter 96® AQueous One Solution Cell Proliferation Assay (MTS) was purchased from Promega (Madison, WI, USA). Triton-X was obtained from Bio-Rad Laboratories (Hercules, CA, USA). Phalloidin-iFluor 488 Reagent (ab176753) was acquired from Abcam® (Cambridge, UK). Adipose-derived Stem Cells (ADSC, PT-5006) were purchased from Lonza Bioscience (Basel, Switzerland). 27G standard conical bioprinting nozzles and 3 mL empty cartridges with end and tip caps were obtained from Cellink (Boston, MA, USA). All other materials obtained were of research grade and used as received.

## 2. Methods

### 2.1. Bioink formulation

Aqueous solutions of gellan gum and MgCl2 (crosslinker for gellan gum) were separately prepared by dissolving their respective powders into double distilled water (ddH2O). All gellan gum solutions were progressively heated to 90°C and kept at this temperature for 30 minutes until a completely homogeneous solution was obtained. After this, gellan gum solutions were cooled to 37°C and maintained at this temperature until further use. A method for stepwise geometric dilution of PGS (PREGEFLO® PI10, a pregelatinized potato starch cross-linked with sodium trimetaphosphate) into the existing gellan gum precursor solution has been developed. Geometric dilution was required as PGS powder tends to clump readily if added in large volumes. Glycerol was included as a hydrophilic plasticizer to decrease the hydrogels' brittleness and make the material ductile as well as stretchable when 3D-printed into the form of a film. The method is illustrated in the **Figure 1.** A glycerol-PGS-gellan gum bioink (bioink-1) was formed with a final concentration of 15% (v/v) glycerol, 0.8% (w/v) gellan gum, and 5% (w/v) PGS.

Type-1 collagen solution was neutralized to a final concentration of 1.0 mg/mL, using 10x PBS, IN NaOH, and ddH2O, according to the manufacturer's protocol. The neutralized collagen was always prepared sterile and kept stable on ice until further use.

ADSC received from Lonza Bioscience were thawed and cultured on Nunc™ T-75 cell culture flasks (Roskilde, Denmark), according to the manufacturer's protocol. All cells were cultured and expanded under basal condition, using 90% (v/v) low-glucose DMEM supplemented with 10% (v/v) FBS and 1% (v/v) penicillin-streptomycin. Culture media were replaced every two - three days. ADSC between passages 3 - 6 were used. All cells were harvested between 80 - 90% confluency. To harvest cells, ADSC were incubated with Accutase® solutions at 37°C in a humidified atmosphere of 5% CO2 in air for 5 minutes. Cell densities were counted using 10 $\mu$L of cell suspension each, with trypan blue staining to distinguish live and dead cells. After centrifugation at 200 x g for 5 minutes, cell pellets were resuspended with appropriate volume of neutralized type-1 collagen to achieve a cell density of $1 \times 10^3$ cells/$\mu$L. The ADSC-laden collagen solution is bioink-2. Cell-unladen collagen solution, at the same concentration, was loaded for 3D-printing of hydrogel films for physical characterization.

### 2.2. 3D-bioprinting

Cellink BioX printer was swabbed down with 70% ethanol and exposed to in-built bactericidal UV light using the "clean" function to ensure sterile printing environment prior to any 3D-printing activity. Next, bioink-1 and bioink-2 solutions were separately transferred into 3 mL empty cartridges from Cellink. Bioink-1 was loaded as printhead-1 while bioink-2 was loaded as printhead-2. A thin film of the dimension 20 mm (L) x 20 mm (W) x 0.2 mm (H) was designed using the computer-aided design (CAD) software AutoCAD® 2021 (Autodesk, Inc., San Rafael, Ca, USA). The design file was loaded as .STL file onto the printing control software of Cellink BioX. The printing parameters for the printheads can be found in **Table 1.**

**Table 1. Printing parameters for 3D-bioprinting of ADSC-laden IPN hydrogels using Cellink BioX**

| Printing parameters | Printhead-1 | Printhead-2 |
|---|---|---|
| Tool Type | Pneumatic 3 mL | |
| Nozzle diameter | 27G | |
| Print pressure | 15 - 20 kPa | 5 - 7 kPa |
| Pre-/ Post-flow times | 0 / 0 milliseconds | |
| Printhead temperature | 37 °C | Off (~ 20 °C) |
| Print-bed temperature | Off (R.T.P.) → turn to 37 °C after printing | |
| Nozzle movement speed | 7 mm/s | 5 mm/s |
| Infill pattern | Grid | |
| Infill density | 10 - 15% | |
| First layer print height | 0.1 - 0.2 mm (66%) | |
| Print pattern | Alternating for both "infill" and "perimeter" tabs | |

*2.3. 3D-printing resolution analysis*

The printing resolution was evaluated using dimensional analysis assay. The bioinks were extruded at 5% infill density to generate two intersecting filaments. The morphology of the filaments was imaged and measured using ImageJ (NIH). The widths of the filaments were expressed as a percentage against the external diameter of the 27G bioprinting nozzle (n = 3). The smaller the deviation of the average percentage from 100%, the higher the print accuracy and resolution.

For LbL 3D-printed geometries, there exists a tendency for the deposited layers to slump. The degree of slumping for the present 3D-printed hydrogel films was assessed in terms of shape fidelity factor (SFF). SFF is defined as the ratio of the area of the 3D-printed films (n = 3), determined using ImageJ, to the CAD model (i.e. 20x20 mm2). The closer the SFF to the value of 1, the lower the tendency of the deposited layers of our 3D-printed hydrogel to slump.

As the 3D-printed hydrogels would be used for 3D culturing of encapsulated ADSC over a period of 21 days, their shape stability in culture media needs to be assessed. Three 3D-printed films (n = 3) were incubated with 5 mL of basal culture media for a duration of 21 days. After the incubation period, SFF values of the hydrogel films were determined and expressed as a percentage against their SFF values obtained immediately after being 3D-printed. The closer the average percentage change in SFF values to 100%, the higher the shape stability of the 3D-printed hydrogel films.

*2.4. Rheometry*

Rheological characterization of hydrogels was performed with an oscillatory rheometer (MCR 302, Antor Paar, Austria), using plate-plate geometry (diameter = 8 mm, working gap = 1 mm). Amplitude sweep and frequency sweep tests were conducted on hydrogels incubated in basal culture media, used for culturing ADSC, to determine if the 3D-printing process altered the rheological properties of the hydrogels significantly. Tests were performed using three different samples (n = 3) and compared between hydrogels fabricated manually (lab) and 3D-printed. Sufficient hydrogels were positioned on the base plate to fill the gap each run. All measurements were conducted at 25°C.

Amplitude sweeps were first conducted to determine the limit of the linear viscoelasticity (LVE). Storage (G') and loss (G") moduli were obtained between the range of 0.1 to 100% shear strain at a constant frequency of 1 Hz. The linearity limit was determined using a straight ruler on the graph plotted. The shear-dependent behavior and inner structure of hydrogels were studied via frequency sweeps. G' and G" moduli measurements were completed from minimum to maximum frequency between 0.1 to 10 rad/s. Shear strain was set to a constant value, below the predetermined LVE prior to initiation of each measurement.

Step-strain rheological measurements were performed at a constant shear frequency of 1 Hz, at an alternating non-deformatory (0.5% shear strain) and deformatory shear forces (500% shear strain) for up to three iterations. A total of 15 measurements were conducted per iteration, with an interval of 10 seconds between the measurements. Complex viscosity values were calculated from an average of three samples (n = 3), and potted against time.

*2.5. Cell viability analysis*

After 3D-bioprinting, the encapsulated ADSC were cultured in 5 mL of basal culture media for 72 hours. After which, spent media in all 35 mm petri dishes were removed and replaced with fresh culture media (n = 3), and MTS solution was added to each dish at a 1:10 (MTS : media) volume ratio. The cell-laden hydrogels were then incubated with the MTS for another 3 hours. After which, 100 $\mu$L of the supernatant mixture was removed from each dish and transferred to a transparent, flat, round-bottom 96-well plate (Greiner Bio-One; flat transparent bottom) for absorbance reading at 490 nm absorbance using the Tecan® Infinite M200 pro plate reader (Tecan, Männedorf, Switzerland) (A). Cell-free 3D-printed hydrogels were treated as "blanks" (Ab). All percentage viabilities were obtained by expressing their blank-corrected absorbance readings against that of lab-fabricated ADSC-laden hydrogels of the same dimensions (Ac) [equation (1)].

$$\% \text{ cell viability} = \frac{A - Ab}{Ac - Ab} \text{ X } 100 - (1)$$

*2. 6. Confocal laser scanning microscopy (CLSM)*

In order to ascertain that 3D-bioprinted ADSC were able to adhere and spread in a 3D fashion within the hydrogel matrix, z-stack confocal imaging of F-actin stained cells were conducted. 3D-bioprinted ADSC-laden hydrogels cultured for 0, 7, and 14 days in each of the Greiner Bio-one 35 mm petri dish (Kremsmünster, Austria). At the end of the incubation period, F-actin staining was conducted according to the manufacturer's protocol. Briefly, encapsulated cells were first fixed with 4% paraformaldehyde (PFA) for 20 minutes, then permeabilised with 0.1% Triton-X for 5 minutes. Subsequently, the permeabilized cells were stained with 1x Phalloidin-iFluor 488 reagent for 60 minutes in the dark. The entire staining procedure was conducted at room temperature conditions. Confocal z-stack images were then captured with Zeiss LSM710 (Oberkochen, Germany) using the 20x/0.8 objective lens, with 488 nm argon laser as the excitation source. Images were processed and exported using Imaris v9.5.3. (Bitplane, Zurich, Switzerland).

## 3. Results and Analysis

### 3.1. Bioink formulation

Step-strain rheological measurements were conducted to evaluate the shear recovery of the composite glycerol-PGS-gellan gum bioink (bioink-1). As shown in **Figure 2,** the bioink exhibited good shear recovery, with a registered viscosity change of ~550 Pa.s within 10s, when subjected up to three iterations of non-deformatory and deformatory shear forces. According to Roquette's prior experience from 3D-printing PREGEFLO® PI10, a minimum of 500 Pa.s. viscosity change is required for satisfactory structural integrity of 3D-printed scaffolds. Therefore, the result indicated that the glycerol-PGS-gellan gum composite bioink could be extruded through the bore of the Cellink BioX extrusion-based 3D-printer under shear stress, and rapidly return to its original viscosity upon deposition on the print bed.

Type-1 collagen has also been developed as a bioink for 3D-bioprinting. Its shear-thinning property is well-documented (Lai G. et al. Int J Biol Macromol; 2008, 42(3):285-91). However, collagen's temperature-dependent gelation requires a minimum of 20- 30 minutes of heating at the physiological temperature of 37°C to form crosslinked networks. Therefore, pure collagen bioinks cannot retain their structural integrity immediately after being extruded and would not perform adequately in strep-strain rheological measurements. On the other hand, due to the viscous nature of bioink-1 as well as its ability for rapid (10 s) viscosity recover, the present strategy to extrude cell-laden collagen into a sufficient viscous bioink-1 offers an excellent strategy to overcome the issue with bioink-2. Without extruding bioink-2 into bioink-1, 3D-bioprinting of bioink-2 cannot be achieved due to complete slumping of the flowing bioink-2 suspension.

### 3.2. Established methodology for 3D-Bioprinting of ADSC-laden IPN hydrogel films

A sterile 35 mm cell culture petri dish was secured onto the printhead of the printing platform and served as the collecting chamber for the 3D-bioprinted hydrogel film. Bioink-1 was extruded to form a viscous film before ADSC-laden bioink-2 was extruded into the viscous liquid to achieve polymer interpenetration. The LbL positioning system of Cellink BioX 3D-printer allowed the nozzles to be sequentially extruded at the same location for IPN to form. The IPN hydrogel film was able to retain its structural integrity both before and after bioink-2 was extruded. There was no observable seepage from the 3D-bioprinted films, indicating high probability of successful polymer interpenetration as well as ADSC encapsulation. After 3D-bioprinting, the temperature on the print bed can be switched to 37°C and left at this temperature for collagen fibrillogenesis to occur over 20 - 30 minutes. After which, $MgCl_2$-enriched cell culture media can be added into the petri dish to crosslink gellan gum and nourish the encapsulated ADSC.

### 3.3. Resolution of 3D-printed gellan gum-collagen IPN hydrogel

#### 3.3.1. Dimensional analysis of 3D-printed bioinks

The average percentage of the widths of 3D-printed filaments is 109.46% of the injector nozzle tip (Table 2). This indicates that the bioinks are of sufficient viscosity to ensure an acceptable printing resolution.

**Table 2.** Dimensional analysis and SFF determination for 3D-printed gellan gum-collagen IPN hydrogel films

| | Diameter of 3D-printing nozzle (mm) (i) | Length of 3D-printed filament (mm) (ii) | Percentage of (ii) / (i) | Average |
|---|---|---|---|---|
| Dimensional analysis (n = 3) | 0.41 | 0.44887 | 109.46% | 109.46% |
| | 0.41 | 0.40799 | 99.51% | |
| | 0.41 | 0.48959 | 119.41% | |

(continued)

| | CAD model film surface area (mm$^2$) (i) | 3D-printed film surface area (mm$^2$) (ii) | Shape fidelity factor (SFF) (ii) / (i) | Average |
|---|---|---|---|---|
| | 400 | 569.633 | 1.4241 | |
| Shape fidelity (n = 3) | 400 | 521.026 | 1.3026 | 1.36 |
| | 400 | 545.830 | 1.3646 | |

3.3.2. Shape fidelity of 3D-printed gellan gum-collagen IPN hydrogel

The average shape fidelity factor (SFF) of the 3D-printed hydrogel film is 1.36 **(Table 2)**. This indicates that the 3D-printed hydrogels did not experience significant slumping given the present LbL 3D-printing protocol. Furthermore, this indicates that the pregelatinized starch has conferred bioink-1 with adequate viscosity, allowing bioink-2 to be extruded into it while retaining structural integrity. Overall, the results from dimensional analysis and SFF determination showed that the present bioinks and 3D-printing methodology are capable of fabricating the IPN hydrogel films.

3.3.3. Shape stability of 3D-printed gellan gum-collagen IPN hydrogel in cell culture media

The average SFF of the 3D-printed films after 21 days of incubation in cell culture media is 1.37. This value is not statistically significantly different from 1.36 (p > 0.05), the SFF of the 3D-printed hydrogel films determined immediately after being fabricated. Visually, the 3D-printed hydrogel films did not appear to have disintegrated or fragmented after the incubation period **(Figure 3)**. This indicates that the present 3D-printed hydrogel films can serve as a robust ADSC scaffold, allowing the cells to stably reside within its matrix for up to 21 days of incubation in basal cell culture media.

*3.4. Rheological properties*

Amplitude sweeps determined the limit of linear viscoelasticity (LVE) of both 3D-printed and manually fabricated hydrogels to be 1.05% shear strain **(Figure 4A),** below which hydrogels are sheared within a non-destructive deformation range. Therefore, 1% shear strain was fixed for subsequent frequency sweeps at 25°C. The frequency sweep results showed that storage moduli (G') of 3D-printed hydrogels were not statistically significantly (p > 0.05) different from that of manually fabricated (lab) hydrogels at the angular frequency of 1 Hz **(Figure 4B).** This suggested that the present 3D-printing methodology was able to fabricate hydrogels of similar stiffness to manually fabricated hydrogels. Given that the stiffness of the hydrogels play an important role in affecting cellular behavior via mechanotransduction, the present 3D-printed IPN hydrogels films can provide as good a cell-conducive environment as manually fabricated IPN hydrogels for cell adhesion, spread, and proliferation.

*3.5. Cell viability of 3D-bioprinted ADSC*

The cell viability of 3D-bioprinted ADSC was determined to be ~77.4% of the cell viability of ADSC encapsulated within manually fabricated hydrogel films **(Figure 5)**. For extrusion-based 3D-bioprinting, the need to expose cells to shear force is known to reduce cell viability by a minimum of ~20% (Khalida Fakhruddin et al 2018. IOP Conf. Ser.: Mater. Sci. Eng. 440 012042). Therefore, as no drastic reduction in cell viability of 3D-bioprinted ADSC was observed, the present 3D-printing methodology is adequately designed for fabricating the ADSC-laden gellan gum-collagen IPN hydrogel films.

*3.6. Cell adhesion, spread, and proliferation of 3D-bioprinted ADSC within the hydrogel matrix*

Three dimensional (3D) confocal images of F-actin stained ADSC were taken to provide visual evidence of their successful cell adhesion and migration within the 3D matrix of the hydrogels. Viable encapsulated ADSC were observed to exist in a rounded morphology immediately after 3D-bioprinting, and transform into an elongated morphology (their normal morphology) with rapidly increasing cell density after 7 and 14 days of culture **(Figure 6)**. This result suggested that 3D-bioprinted ADSC retained their basic cellular functions of cell adhesion, proliferation, and migration.

**Claims**

1. A method for preparing cell-laden collagen- gellan gum interpenetrating network (IPN) hydrogel by 3D bioprinting, the method comprising:

   a) preparing a first bioink by dissolving gellan gum with a viscosity enhancer in a solvent, preferably by heating the solution at a temperature above hydration temperature and by lowering the temperature,
   b) preparing a second bioink by suspending stem cells in collagen solution, preferably in neutralized type I collagen solution,
   c) depositing the first bioink on a support media,
   d) depositing the second bioink into the first bioink,
   e) heating the bioprinted product obtained from the deposition of the first and second bioinks of step c) and d) to achieve collagen fibrillogenesis,
   f) adding an ionic crosslinking agent, preferably $MgCl_2$ solution to cross-link gellan gum to obtain a cell-laden collagen-gellan gum IPN hydrogel.

2. The method of claim 1 wherein said first bioink comprises between 0.1 and 10 % (w/v), preferably about 0.8% (w/v) of gellan gum.

3. The method of claim 1 or 2 wherein said viscosity enhancer is a pregelatinized starch, preferably a pregelatinized starch derived from potato.

4. The method of claim 3 wherein said first bioink comprises between 1 and 20 % (w/v), more preferably about 5% (w/v) of a pregelatinized starch, preferably derived from potato.

5. The method of claim 3 or 4 wherein said pregelatinized starch is crosslinked, preferably with sodium trimetaphosphate.

6. The method according to any one of claims 1 to 5 wherein said first bioink comprises glycerol preferably between 1 and 30 % (v/v) glycerol, more preferably about 15% (v/v) glycerol.

7. The method according to any one of claims 1 to 6 wherein said second bioink comprises less than 0.2 mg/mL of collagen.

8. The method according of any one of claims 1 to 7 wherein said fibrillogenesis of collagen is achieved by heating the bioprinted product to 37°c or above.

9. The method according to any one of claims 1 to 8 wherein said stem cells are adipose derived stem cells.

10. A 3D bioprinted cell-laden collagen-gellan gum IPN hydrogel comprising a viscosity enhancer such as a pregelatinized starch derived from potato, preferably crosslinked with sodium trimetaphosphate, more preferably comprising between 1 and 20 % (w/v), again more preferably about 2.5% (w/v) of a pregelatinized starch.

11. The 3D bioprinted hydrogel of claim 10 comprising between 0.1 and 10 % (w/v), more preferably about 0.4% (w/v) of gellan gum.

12. The 3D bioprinted hydrogel film of claim 10 or 11 comprising glycerol preferably between 1 and 30 % (v/v) glycerol, more preferably about 7.5% (v/v) glycerol.

13. The 3D printed hydrogel film glycerol according to any one of claims 10 to 12 comprising less than 0.2 mg/mL of collagen.

14. An artificial skin graft comprising the 3D printed hydrogel film according to any one of claims 10 to 13.

15. A kit comprising a first bioink comprising gellan gum and a viscosity enhancer and a second bioink comprising cell laden collagen solution.

Pre-heat 42.5mL of water + 7.5mL of glycerol to 90 °C

Transfer 400 mg of gellan gum + stir @400rpm for 5 mins

Transfer small portions of 2.5 g PGF & stir each time to ensure dissolution

Maintain @90 °C + stir for at least 30 mins

Transfer into 50 mL Falcon tubes and leave formulation to set @R.T.P. overnight

**FIG. 1**

EP 4 098 286 A1

**FIG. 2**

**FIG. 3**

FIG. 4

**FIG. 5**

FIG. 6

Europäisches
Patentamt

European
Patent Office

Office européen
des brevets

# EUROPEAN SEARCH REPORT

Application Number

EP 21 30 5722

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/069964 A1 (BOYER CHRISTEN J [US] ET AL) 11 March 2021 (2021-03-11) | 10-15 | INV.<br>A61L27/24<br>A61L27/36 |
| Y | * paragraphs [0106] - [0108], [0113], [0117] - [0125]; claims 1-20; examples 1-3; table 1 * | 1-9 | A61L27/38<br>A61L27/60<br>B33Y10/00<br>B33Y80/00 |
| X | US 2016/367358 A1 (TRAN BAO [US]) 22 December 2016 (2016-12-22)<br>* paragraphs [0076], [0078], [0099]; claims 1-20 * | 10-14 | |
| Y | CHEN HONG ET AL: "Bone Marrow-Derived Mesenchymal Stem Cells Encapsulated in Functionalized Gellan Gum/Collagen Hydrogel for Effective Vascularization", ACS APPLIED BIO MATERIALS, [Online] vol. 1, no. 5, 19 November 2018 (2018-11-19), pages 1408-1415, XP055849299, US ISSN: 2576-6422, DOI: 10.1021/acsabm.8b00361 https://pubs.acs.org/doi/pdf/10.1021/acsabm.8b00361 [retrieved on 2021-11-15] * the whole document * | 1-9 | |
| | | | **TECHNICAL FIELDS SEARCHED (IPC)**<br><br>A61L<br>B33Y |
| A | DEEPAK GUPTA ET AL: "Multiscale porosity in a 3D printed gellan-gelatin composite for bone tissue engineering", BIOMEDICAL MATERIALS, INSTITUTE OF PHYSICS PUBLISHING, BRISTOL, GB, vol. 16, no. 3, 16 April 2021 (2021-04-16), page 34103, XP020366011, ISSN: 1748-605X, DOI: 10.1088/1748-605X/ABF1A7 [retrieved on 2021-04-16] * the whole document *<br><br>-/-- | 1-15 | |

The present search report has been drawn up for all claims

| Place of search | Date of completion of the search | Examiner |
|---|---|---|
| The Hague | 16 November 2021 | Burtan, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 21 30 5722

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Online Research ET AL: "University of Wollongong Gelatin and gellan gum based hydrogel materials: towards soft tissue scaffolds", Thesis, 1 January 2013 (2013-01-01), XP055849243, Retrieved from the Internet: URL:https://ro.uow.edu.au/cgi/viewcontent.cgi?referer=https://scholar.google.com/&httpsredir=1&article=5131&context=theses [retrieved on 2021-10-08] * the whole document * | 1-15 | |
| A | US 2016/368211 A1 (TRAN BAO [US]) 22 December 2016 (2016-12-22) * claims 1-20 * | 1-15 | |
| A | LI JINHUA ET AL: "3D printing of hydrogels: Rational design strategies and emerging biomedical applications", MATERIALS SCIENCE AND ENGINEERING: R: REPORTS, ELSEVIER, AMSTERDAM, NL, vol. 140, 18 February 2020 (2020-02-18), XP086123009, ISSN: 0927-796X, DOI: 10.1016/J.MSER.2020.100543 [retrieved on 2020-02-18] * the whole document * | 1-15 | TECHNICAL FIELDS SEARCHED (IPC) |

The present search report has been drawn up for all claims

| Place of search | Date of completion of the search | Examiner |
|---|---|---|
| The Hague | 16 November 2021 | Burtan, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 21 30 5722

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2021

| Patent document cited in search report | | Publication date | Patent family member(s) | | Publication date |
|---|---|---|---|---|---|
| US 2021069964 | A1 | 11-03-2021 | NONE | | |
| US 2016367358 | A1 | 22-12-2016 | US 2016367358 A1 | | 22-12-2016 |
| | | | US 2018221135 A1 | | 09-08-2018 |
| | | | US 2020054440 A1 | | 20-02-2020 |
| US 2016368211 | A1 | 22-12-2016 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020225336 A

**Non-patent literature cited in the description**

- **PRZEKORA A; et al.** *Cells,* 2020, vol. 9 (7), 1622
- **MOORHOUSE R. et al.** *ACS symposium Series,* 1981, (150), 111
- *CHEMICAL ABSTRACTS,* 55963-33-2
- **LAI G. et al.** *Int J Biol Macromol,* 2008, vol. 42 (3), 285-91
- **KHALIDA FAKHRUDDIN et al.** *IOP Conf. Ser.: Mater. Sci. Eng.,* 2018, vol. 440, 012042